# EUROPEAN PATENT SPECIFICATION

(11) **EP 2 433 979 B1**
(45) Date of publication and mention of the grant of the patent: **12.06.2013**
(21) Application number: 10777657.7
(22) Date of filing: 27.04.2010
(51) Int. Cl.: C08J 3/12, C08G 77/04, C08G 77/06, C08L 101/00, A61K 8/89, A61K 8/11, A61K 8/893, A61Q 19/00, A61K 8/02

(54) **IRREGULAR-SHAPED HOLLOW MICROPARTICLE, METHOD FOR PRODUCING SAME, AND COSMETIC MATERIAL AND RESIN COMPOSITION CONTAINING IRREGULAR-SHAPED HOLLOW MICROPARTICLE**
IRREGULÄR GEFORMTES HOHLES MIKROPARTIKEL, VERFAHREN ZU SEINER HERSTELLUNG SOWIE KOSMETIKMATERIAL UND HARZZUSAMMENSETZUNG MIT DEM IRREGULÄR GEFORMTEN HOHEN MIKROPARTIKEL
MICROPARTICULE CREUSE DE FORME IRRÉGULIÈRE, SON PROCÉDÉ DE PRODUCTION, ET MATÉRIAU COSMÉTIQUE ET COMPOSITION DE RÉSINE CONTENANT UNE MICROPARTICULE CREUSE DE FORME IRRÉGULIÈRE

(30) Priority: 21.05.2009 JP 2009123380; 07.04.2010 JP 2010088277
(43) Date of publication of application: 28.03.2012
(73) Proprietor: Takemoto Yushi Kabushiki Kaisha, Gamagori-shi, Aichi-ken 443-8611 (JP)
(72) Inventor: ARATANI Satoshi, Gamagori-shi Aichi 443-8611 (JP); ISHIKAWA Fumiyoshi, Gamagori-shi Aichi 443-8611 (JP); SAITO Chiaki, Gamagori-shi Aichi 443-8611 (JP)
(74) Representative: Wallace, Sheila Jane
(86) International application number: PCT/JP2010/057419
(87) International publication number: WO 2010/134422

(56) References cited:
- WO-A1-2006/075711
- JP-A- 6 172 125
- JP-A- 10 218 950
- JP-A- 61 225 254
- JP-A- 2000 191 788
- JP-A- 2000 191 789
- JP-A- 2002 179 708
- JP-A- 2003 171 465

## Description

### Background of the Invention

This invention relates to non-spherical hollow fine particles, as well as methods of their production and their use. Fine particles of various substances have been in use in many applications. Their shapes are mostly indefinite, and fine particles of each type have been playing their suitable roles as an industrial material. In recent years, however, as the characteristics required of them in various applications become highly advanced, there are beginning to appear many situations where fine particles with controlled shapes are desired. As examples, improvements in the optical characteristics in the field of display devices and optical diffusers, miniaturization in size in the field of electronic components, and improvements in usability of cosmetic products may be considered. This invention relates to non-spherical hollow fine particles, as well as method of their production and their use.

There have been many proposed ideas regarding both inorganic and organic fine particles with controlled shapes. As for organic fine particles, Japanese Patent Publications Tokkai 09-103804 and 11-292907, for example, considered polystyrene fine particles, Japanese Patent Publication Tokkai 11-116649, for example, considered polyurethane fine particles, Japanese Patent Publication Tokkai 11-140181, for example, considered polyimide fine particles, and Japanese Patent Publication Tokkai 61-159427, for example, considered organosilicone fine particles. Since almost all of these prior art organic fine particles are spherical or nearly spherical, there have in recent years been an increasing number of situations wherein these prior fine particles cannot respond to the highly advanced requirements imposed upon them for purposes of use. In view of the above, as examples of organic fine particles with modified shapes, Japanese Patent Publication Tokkai 07-157672, for example, proposed hollow organic fine particles having large protrusions and indentations, Japanese Patent Publication Tokkai 2000-191788, for example, proposed organic fine particles having a plurality of small indentations on the surface, Japanese Patent Publication Tokkai 2003-171465, for example, proposed organic fine particles shaped like a rugby ball, and Japanese Patent Publication Tokkai 2003-128788, for example, proposed semispherical organic fine particles. Even such prior art organic fine particles with modified shapes have problems of not being able to fully respond to the highly advanced requirements of recent years imposed on them for purposes of use.

### Summary of the Invention

It is therefore an object of this invention to provide non-spherical hollow fine particles which will be able to respond to the highly advanced requirements of recent years imposed on them for purposes of actual use, including further improvements in optical characteristics such as total light transmittance and optical diffusibility and in feeling, soft focus and durability related to cosmetic products, as well as methods of their production and their use.

The inventors herein have carried out investigations in order to solve the aforementioned problems and discovered as a result thereof that what are suitable are non-spherical hollow fine particles of a specific kind having a plurality of concave parts of a specific size on the surface, a hollow part inside connected to the surface, and a spindle shape as a whole.

Thus, this invention relates to non-spherical hollow fine particles characterized as each having a spindle shape as a whole with a major axis and a minor axis, a plurality of concave parts 21 on the surface 11, a hollow part 41 inside connected to the surface 11 through a crack 51 extending along the major axis L₁, and, the average length of the major axes being 0.1-30µm and the ratio of the average length of the minor axes L₂ to the average length of the major axes L₁ being 0.3-0.8. The invention also relates to methods of producing such non-spherical hollow fine particles, as well as cosmetic products and resin compositions using such non-spherical hollow fine particles.

### Brief Description of the Drawings

Fig. 1 is an enlarged approximate front view of a non-spherical hollow fine particle embodying this invention.
Fig. 2 is an enlarged plan view of the non-spherical hollow fine particle shown in Fig. 1.
Fig. 3 is a sectional view seen along line 3-3 in Fig. 2.
Fig. 4 is a scanning electron microscopic photograph with magnification 4000 of a non-spherical hollow fine particle embodying this invention.
Fig. 5 is a scanning electron microscopic photograph with magnification 10000, showing a sectional view of a non-spherical hollow fine particle embodying this invention in the direction of its minor axis.

### Detailed Description of the Invention

Non-spherical hollow fine particles according to this invention are explained first. Non-spherical hollow fine particles according to this invention each have a plurality of concave parts 21 on its surface 11, a hollow part 41 in its interior 31 connected to the surface 11 through a crack 51 extending in the direction of its major axis L₁, and a spindle shape as a whole. As non-spherical hollow fine particles according to this invention, those comprising a polysiloxane cross-link structure are more useful and preferable for the purpose of their use. This polysiloxane cross-link structure is a structure having siloxane units forming a three-dimensional network structure. Although there are no specific limitations regarding the kind and ratio of the siloxane units forming the polysiloxane cross-link structure, those comprising siloxane units shown by SiO₂, siloxane units shown by R¹SiO_{1.5}, and siloxane units shown by R²R³SiO, where R¹, R² and R³ are each an organic group having a carbon atom directly connected to a silicon atom, are preferred.

Examples of R¹, R² and R³ include organic groups with 1-12 carbon atoms such as alkyl group, cycloalkyl group, aryl group, alkylaryl group and aralkyl group but alkyl groups with 1-4 carbon atoms such as methyl group, ethyl group, propyl group and butyl group and phenyl group are preferable and methyl group is even more preferable. When R¹, R² and R³ are such organic groups, preferable examples of siloxane units R¹SiO_{1.5} and R²R³SiO include methyl siloxane unit, ethyl siloxane unit, propyl siloxane unit, butyl siloxane unit and phenyl siloxane unit.

When the polysiloxane cross-link structure is formed with such siloxane units, there is no specific limitation on the ratio among these siloxane units but it is preferable to have siloxane units SiO₂ in an amount of 20-45 molar %, siloxane units R¹SiO_{1.5} in an mount of 50-75 molar % and siloxane units R²R³SiO in an amount of 5-27 molar % such that the total would be 100 molar %.

As explained above, non-spherical hollow fine particles according to this invention are characterized as each having a plurality of concave parts 21 on its surface 11, a hollow part 41 in its interior 31 connected to the surface 11 through a crack 51 extending in the direction of the major axis L₁, and a spindle shape as a whole. Furthermore, the average length of the major axes L₁ is in the range of 0.1-30µm and the ratio of the average length of the minor axes L₂ to the average length of the major axes L₁ is in the range of 0.3-0.8. Preferably, however, the average length of the major axes L₁ is in the range of 0.5-20µm.

Non-spherical hollow fine particles according to this invention each have a crack 51 in the direction of the major axis L₁. This crack 51 may be substantially closed, evidently open or a mixture of both, and is serviceable in all these forms. This crack 51 is connected to the hollow part 41. Although there is no particular limitation on the size of this hollow part 41, its existence is important for having the desired effects of this invention.

Non-spherical hollow fine particles according to this invention each have a plurality of concave parts 21 on the surface 11. There is no particular limitation on the size of such concave parts 21 but the ratio of the average of their maximum diameters m₁ to the average of their major axes L₁ is preferably in the range of 0.01 - 0.30. The existence of such concave parts is important for having the desired effects of this invention.

Regarding the non-spherical hollow fine particles of this invention, the average of their major axes L₁, the average of their minor axes L₂ and the average of the maximum diameter m₁ of their concave parts 21 are all averages of values obtained by measuring on 20 arbitrarily selected particles on a scanning electron microscopic image.

One of the indicators of the characteristics of the non-spherical hollow fine particles of this invention is the magnitude of oil absorption. There is no particular limitation on such oil absorption, it is preferably in the range of 50-150ml/100g.

Next, a method of producing non-spherical hollow fine particles according to this invention will be described. Non-spherical hollow fine particles according to this invention can be obtained by using silanol group forming silicide SiX₄ in an amount of 20-45 molar %, silanol group forming silicide R⁴SiY₃ in an amount of 50-75 molar % and silanol group forming silicide R⁵R⁶SiZ₂ in an amount of 5-27 molar % such that the total would be 100 molar %, where R⁴, R⁵ and R⁶ are each an organic group having a carbon atom directly connected to a silicon atom, and X, Y and Z are each alkoxy group with 1-4 carbon atoms, alkoxyethoxy group having alkoxy group with 1-4 carbon atoms, acyloxy group with 2-4 carbon atoms, N,N-dialkylamino group having alkyl group with 1-4 carbon atoms, hydroxyl group, halogen atom or hydrogen atom, obtained by firstly generating silanol compounds by causing silanol group forming silicide SiX₄ to contact water in the presence of a basic catalyst for hydrolysis and then causing a condensation reaction of these silanol compounds with silanol group forming silicides R⁴SiY₃ and R⁵R⁶SiZ₂ in an aqueous condition with the presence of a basic catalyst and a cationic surfactant.

Examples of R⁴, R⁵ and R⁶ include organic groups without reactivity such as alkyl group; phenyl group, and alkoxyalkyl group and organic groups with reactivity such as epoxy group vinyl group, (meth)acryloylalkyl group and mercapto group.

It is preferable from the points of view of ease in obtaining and stability in production if R⁴, R⁵ and R⁶ are each alkyl group with 1-4 carbon atoms or phenyl group.

Silanol group forming silicide SiX₄ is a compound which eventually forms siloxane unit SiO₂. Examples of X in SiX₄ include (1) alkoxy groups with 1-4 carbon atoms such as methoxy group and ethoxy group, (2) alkoxyetoxy groups having alkoxy group with 1-4 carbon atoms such as methoxyethoxy group and butoxyethoxy group, (3) acyloxy groups with 2-4 carbon atoms such as acetoxy group and propyoxy group, (4) N,N-dialkylamino groups having alkyl group with 1-4 carbon atoms such as dimethylamino group and diethylamino group, (5) hydroxyl group, (6) halogen atoms such as chlorine atom and bromine atom, and (7) hydrogen atom.

Examples of silanol group forming silicide SiX₄ include tetramethoxy silane, tetraethoxy silane, tetrabutoxy silane, trimethoxyethoxy silane, tributoxyethoxy silane, tetraacetoxy silane, tetrapropyoxy silane, tetra(dimethylamino) silane, tetrahydroxy silane, tetrachloro silane, and chlorotrihydrogen silane, among which tetramethoxy silane, tetraethoxy silane and tetrabutoxy silane are preferred.

Silanol group forming silicide R⁴SiY₃ is a compound which eventually forms siloxane units R¹SiO_{1.5}. Y in R⁴SiY₃ is similar to X in SiX₄ and R⁴ in R⁴SiY₃ is similar to R¹ in R¹SiO_{1.5}.

Examples of silanol group forming silicide R⁴SiY₃ include methyltrimethoxy silane, ethyltriethoxy silane, propyltributoxy silane, butyltributoxy silane, phenyltris(2-methoxyethoxy)silane, methyltris(2-butoxyethoxy)silane, methyltriacetoxysilane, methyltripropyoxy silane, methyl silane triol, methylchloro disilanol, methyltrichlorosilane, and methyltrihydrogen silane, among which, as explained above regarding R¹ in siloxane units R¹SiO_{1.5}, those silanol group forming silicides which eventually form methyl siloxane unit, ethyl siloxane unit, propyl siloxane unit, butyl siloxane unit, or phenyl siloxane unit are preferred and those silanol group forming silicides which come to form methyl siloxne are more preferred.

Silanol group forming silicide R⁵R⁶SiZ₂ is a compound which eventually forms siloxane units R²R³SiO. Z in R⁵R⁶SiZ₂ is similar to X in SiX₄ and R⁵ and R⁶ in R⁵R⁶SiZ₂ are similar to R² and R³ in R²R³SiO.

Examples of silanol group forming silicide R⁵R⁶SiZ₂ include dimethyldimethoxy silane, diethyldiethoxy silane, dipropyldibutoxy silane, dibutyldimethoxy silane, methylphenyl methoxyethoxy silane, dimethylbutoxyethoxy silane, dimethyldiacetoxy silane, dimethyldipropyoxy silane, dimethyldi(dimethylamino)silane, dimethyldi(diethylamino)silane, dimethyl silane diol, dimethylchloro silanol, dimethyldicholo silane, and dimethyldihydrogen silane, among which, as explained above regarding R² and R³ in siloxane units R²R³SiO, those silanol group forming silicides which eventually form dimethyl siloxane unit, diethyl siloxane unit, dipropyl siloxane unit, dibutyl siloxane unit or methylphenyl siloxane unit are preferred and those silanol group forming silicides which come to form dimethylsiloxane unit are more preferred.

For producing non-spherical hollow fine particles embodying this invention, silanol group forming silicide SiX₄ in an amount of 20-45 molar %, silanol group forming silicide R⁴SiY₃ in an amount of 50-75 molar % and silanol group forming silicide R⁵R⁶SiZ₂ in an amount of 5-27 molar % are used such that their total would be 100 molar %, silanol group forming silicide SiX₄ being firstly caused to undergo hydrolysis by contacting water in the presence of a basic catalyst so as to produce a silanol compound. A known kind of basic catalyst may be employed for the hydrolysis. Examples of such a basic catalyst include inorganic bases such as sodium hydroxide, potassium hydroxide, sodium bicarbonate, and ammonia and organic bases such as trimethylamine, triethylamine, tetraethyl ammonium hydroxide, dodecyldimethyl hydroxylethyl ammonium hydroxide, and sodium methoxide. It is generally preferable that the catalyst to be made present for the hydrolysis be at a concentration of 0.001-0.5 mass % with respect to the total silanol group forming silicides used in the reaction.

Next, the silanol compound generated as explained above and silanol group forming silicides R⁴SiY₃ and R⁵R⁶SiZ₂ are caused to undergo a condensation reaction in an aqueous condition in the presence of a basic catalyst and a cationic surfactant. As the basic catalyst for the condensation reaction, as in the case of that for the hydrolysis, those of a known kind can be use, and it is preferable to cause it to be present at a concentration of 0.001-0.5 mass % with respect to the total amount of the silanol group forming silicides used for the reaction.

As the cationic surfactant to be added to the reacting system together with the basic catalyst, too, those of a known kind may be used. Examples of such cationic surfactant include quaternary ammonium salts such as lauryl trimethyl ammonium ethosulfate, tributylmethyl ammonium, tetrabutyl ammonium, trimethyloctyl ammonium, trimethyllauryl ammonium, and trimethyloleyl ammonium, and cationic surfactants such as 2-heptadecenyl-hydroxyethylimidazoline. It is normally preferable to cause the cationic surfactant to be made present at a concentration of 0.001-0.55 mass % with respect to the total amount of the silanol group forming silicides used for the reaction.

The mass ratio of water to the total amount of the silanol group forming silicides is normally 10/90 - 70/30. The amount of the catalyst to be used varies according to its kind as well as to the kind of the silanol group forming silicide but it is preferably 1 mass % or less with respect to the total amount of the silanol group forming silicides. The reaction temperature is usually 0 - 40°C but preferably 30°C or less in order to avoid any instantly occurring condensation reaction of the silanol which has been generated by the hydrolysis.

The reaction liquid containing the silanol compounds which has been generated as described above is provided continuously to the condensation reaction to generate organic silicone fine particles that are hollow and of a spindle shape as a whole. By the production method according to the present invention, since the catalyst for the hydrolysis can be used also as the catalyst for the condensation reaction, organic silicone fine particles can be obtained as an aqueous suspension by causing the reaction liquid containing the silanol compounds generated by the hydrolysis to continue undergoing the condensation reaction either as it is or by further adding a catalyst and by heating it to 30 - 80°C. In the production method of this invention, it is desirable to adjust the pH value of this aqueous suspension to 8-10 by an alkali such as ammonia, sodium hydroxide or potassium hydroxide. It is also preferable to adjust the solid concentration of the organic silicone within such an aqueous suspension to 2-20 mass % and more preferably to 5-15 mass % by varying the amount of water to be used.

Non-spherical hollow fine particles of this invention can be used as an aqueous material with the solid component adjusted to be 30-70 mass % by separating from the aforementioned aqueous suspension, say, by passing through a metallic net and through centrifugation or pressure filtration, or they may be used in a dried form. The dried form can be obtained by passing the aqueous suspension through a metallic net, dehydrating by centrifugation or pressure filtration and drying the dehydrated product by heating at 100-250°C. It can also be obtained by a method of directly heating and drying the aqueous suspension by a spray drier at 100-250°C. Such dried materials are preferably crushed, for example, by using a jet mill.

Non-spherical hollow fine particles of this invention thus obtained have a plurality of concave parts 21 on the surface, a hollow part 41 in the interior 31 connected to the surface 11 through a crack 51 extending in the direction of the major axis L₁, and a spindle shape as a whole, the average length of the major axis L₁ being 0.1-30µm and the ratio of the average of the minor axis L₂ to the average of the major axis L₁ being 0.3 - 0.8.

Lastly, cosmetic materials and resin compositions according to this invention are explained. Cosmetic materials according to this invention are characterized as containing those non-spherical hollow fine particles of this invention described above.

Cosmetic materials according to this invention are superior in terms of their soft focus effect with reduced roughness and glare and spread on and fitness to the skin due to their superior optical chatacteristics and high oil absorption when used as a basic cosmetic article in a cream or press form or as an ingredient of a make-up cosmetic article and hence are useful against falling make-up due to sebum. The amount of cosmetic material of this invention to be used should be appropriately selected according to the form of its use but it is preferably 0.5 -50 mass % in the case of a press-form make-up cosmetic material and 0.1 - 30 mass % in the case of a liquid form make-up cosmetic material.

Other materials that can be used together with a cosmetic material of this invention include body pigments, white pigments, pearl pigments, color pigments (dyes), binding ointments, water, surfactants, thickeners, preservatives, antioxidants, and perfumes. Desired cosmetic products can be prepared by any known method for uniformly dispersing such other materials together with a cosmetic material of this invention.

Resin compositions according to this invention are characterized as containing non-spherical hollow fine particles of this invention described above and are useful for improving characteristics of various molded resin products obtained therefrom. In the case of molded resin products requiring advanced optical characteristics such as illumination and display devices, for example, products with high optical transmissivity and haze and improved optical diffusibility are becoming desired due to the requirement for highly effective use of light. Resin compositions according to this invention are useful for obtaining molded resin products satisfying such requirement. For producing molded resin products as described above from a resin composition of this invention, the amount of non-spherical hollow fine particles of this invention to be used is preferably 0.1-5 mass % with respect to the molded resin product. When a resin composition of this invention is used for coating the surface of a separately produced molded resin product, however, they may be contained at a rate of up to 30 mass % in the resin composition as long as allowed by the strength of the coating film.

The present invention, as described above, can sufficiently respond to the requirements of recent years such as further improvement in optical characteristics such as total light transmittance and optical diffusibility regarding molded resin products and further improvement in feeling, soft focus and durability regarding cosmetic products.

Next, the invention will be described in terms of test examples but they are not intended to limit the scope of the invention. In the following test examples and comparison examples, "part" will mean "mass part" and "%" will mean "mass %".

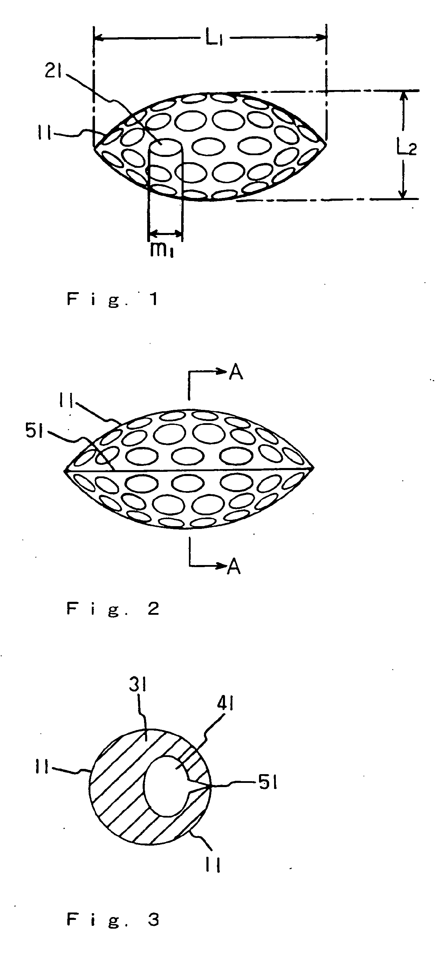

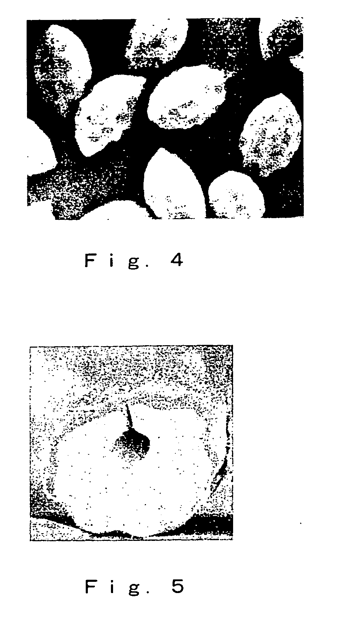

A non-spherical hollow fine particle of this invention schematically shown in Figs. 1-3 has a plurality of concave parts 21 on its surface 11, a hollow part 41 in its interior 31 connected to the surface 11 through a crack 51 extending in the direction of its major axis L₁, and a spindle shape as a whole, the average length of the major axis L₁ being 0.5-20µm and the ratio of the average of the minor axis L₂ to the average of the major axis L₁ being 0.3 - 0.8, and the ratio of the average of the maximum diameter m₁ of the concave parts 21 to the average of the major axis L₁ being 0.01 - 0.30. Such a non-spherical hollow fine particle actually has a shape as illustrated by the scanning electron microscopic photograph of Fig. 4, and its sectional surface along its minor axis L₂ is structured as illustrated by the scanning electron microscopic photograph of Fig. 5.

### Part 1: Synthesis of non-spherical hollow fine particles

### Test Example 1 (Synthesis of non-spherical hollow fine particles (P-1))

Ion exchange water 2000g was taken into a reactor vessel and 30% sodium hydroxide 0.11g was added thereinto and dissolved. Tetraethoxy silane 199.7g (0.96 mols) was further added to carry out hydrolysis with stirring at 15°C for 60 minutes. An aqueous solution was separately prepared in another reactor vessel by dissolving lauryl trimethyl ammonium ethosulfate 0.7g and 30% sodium hydroxide 2.68g in ion exchange water 350g and cooled to 10°C, and the aforementioned hydrolysate solution adjusted to the same temperature was gradually dropped into it with stirring. Dimethyldimethoxy silane 93.6g (0.78 mols) and methyltrimethoxy silane 267.9g (1.97 moles) were further added and the whole was left quietly for one hour while being maintained below 30°C. After it was maintained at the same temperature for 4 hours, it was heated to 60°C for a reaction at the same temperature for 5 hours to obtain a white suspension. After the suspension thus obtained was maintained quietly overnight, the white solid phase obtained by removing the liquid phase by decantation was washed with water by a usual method and dried to obtain non-spherical hollow fine particles (P-1). Regarding non-spherical hollow fine particles (P-1), observations and measurements of the surfaces and observations of the sections of the fine particles, elemental analysis, inductively coupled plasma spectrometry, FT-IR spectrometry and NMR spectrometry were carried out. As a result, it was ascertained that non-spherical hollow fine particles (P-1) were non-spherical hollow fine particles having a plurality of concave parts 21 on the surface 11, a hollow part 41 in the interior 31 connected to the surface 11 through a crack 51 extending in the direction of the major axis L₁, and a spindle shape as a whole, the average length of the major axis being 7.6µm and the ratio of the average of the minor axis L₂ to the average of the major axis L₁ being 0.55, and their structural units being organic silicone fine particles having siloxane units SiO₂ in the amount of 26 molar %, siloxane units R¹SiO_{1.5} in the amount of 53 molar % and siloxane units R²R³SiO in the amount of 21 molar % such that they are together 100 molar %.

### Shapes, average of major axes L₁, average of minor axes L₂, and average of maximum diameters m₁ of the concave parts of non-spherical hollow fine particles (P-1)

A scanning electron microscope (SEMEDX Type N, produced by Hitachi, Ltd.) was used to observe at magnifications of 2000 - 5000 to obtain an SEM image. Arbitrarily selected 20 non-spherical hollow fine particles (P-1) out of this SEM image and observed, their major axes L₁, their minor axes L₂ and the maximum diameters m₁ of the concave parts 21 on their surfaces 11 were actually measured, and their average values were obtained.

### Examination of the hollow parts of non-spherical hollow fine particles (P-1)

FIB (FB-2100 type focusing ion beam fabrication inspection device) was used to carry out cross-sectional surfacing of the non-spherical hollow fine particles. The obtained cross-sectional surfaces of the non-spherical hollow fine particles were observed at magnifications of 8000 - 15000 by using a scanning electron microscope (S-4800 type produced by HITACHI, Ltd.) and SEM images were obtained. The presence or absence of hollow parts of the non-spherical hollow fine particles was examined from these SEM images.

### Measurements of oil absorption by non-spherical hollow fine particles (P-1)

Measurements were made according to JIS-K5101-13-1(2004).

### Analysis of combining siloxane units of non-spherical hollow fine particles (P-1)

Non-spherical hollow fine particles (P-1) 5g were accurately measured and added to 0.05N aqueous solution of sodium hydroxide 250ml to extract all of the hydrolyzable groups in the non-spherical hollow fine particles. Non-spherical hollow fine particles were separated by ultra-centrifugation from the extraction-processed liquid, and after the separated non-spherical hollow fine particles were washed with water and dried at 200°C for 5 hours, elemental analysis, inductively coupled plasma spectrometry and FT-IR spectrometry were carried out on them to measure total carbon content and the amount of contained silicon, and silicon-carbon bonding and silicon-oxygen-silicon bonding were examined. Based on these analyzed values, integrated values of NMR spectrum of CP/MAS on solid ²⁹Si, the number of carbon atoms in R⁴ of silanol group forming silicide R⁴SiY₃, and the numbers of carbon atoms in R⁵ and R⁶ of silanol group forming silicide R⁵R⁶SiZ₂, the ratios of siloxane units SiO₂, siloxane units R¹SiO_{1.5} and siloxane units R²R³SiO were calculated.

### Test Examples 2-7 (Syntheses of non-spherical hollow fine particles (P-2) - (P-7))

Non-spherical hollow fine particles (P-2) - (P-7) were synthesized as done in Test Example 1 and observations, measurements and analyses similar to those done in Test Example 1 were carried out.

### Comparison Example 1 (Synthesis of fine particles (R-1)

Ion exchange water 100g, acetic acid 0.01g and 10% aqueous solution of dodecylbenzene sodium sulfonate 1.8g were taken into a reactor vessel and made into a uniform aqueous solution. Tetraethoxy silane 56.2g (0.27 mols), methyltrimethoxy silane 74.8g (0.55 mols) and dimethyldimethoxy silane 21.6g (0.18mol) were added to this aqueous solution to carry out hydrolysis at 30°C for 30 minutes. Next, ion exchange water 700g and 30% aqueous solution of sodium hydroxide 0.48g were added into another reactor vessel to prepare a uniform aqueous solution. While this aqueous solution was being stirred, the aforementioned hydrolyzed liquid was gradually added to carry out a reaction at 15°C for 5 hours and further for 5 hours at 80°C to obtain a suspension. After this suspension was left quietly overnight, its liquid phase was removed by decantation, the white solid phase thus obtained was washed with water by a usual method and dried to obtain fine particles (R-1). Observations, measurements and analyses similar to those in Test Example 1 were carried out on fine particles (R-1). Details of non-spherical hollow fine particles, etc. of the examples synthesized as above are shown together in Tables 1-3.

**Table 1**

| | Type of particles | Silanol group forming silicide SiX₄ | Silanol group forming silicide R⁴SiY₃ | Silanol group forming silicide R⁵R⁶SiZ₂ | Catalyst for hydrolysis | Catalyst for condensation reaction | Surfactant |
|---|---|---|---|---|---|---|---|
| TE-1 | P-1 | SM-1 / 26 | SM-3 / 53 | SM-6 / 21 | CA-1 / 0.017 | CA-1 / 0.143 | C-1 / 0.124 |
| TE-2 | P-2 | SM-1 / 32 | SM-3 / 54 | SM-6 / 14 | CA-1 / 0.075 | CA-1 / 0.066 | C-1 / 0.035 |
| TE-3 | P-3 | SM-1 / 22 | SM-3 / 62 | SM-6 / 6 | CA-2 / 0.022 | CA-2 / 0.210 | C-2 / 0.008 |
| | | | SM-4 / 10 | | | | |
| TE-4 | P-4 | SM-2 / 40 | SM-3 / 44 | SM-6 / 8 | CA-2 / 0.035 | CA-2 / 0.115 | C-2 / 0.016 |
| | | | SM-5 / 8 | | | | |
| TE-5 | P-5 | SM-2 / 37 | SM-3 / 51 | SM-7 / 12 | CA-1 / 0.008 | CA-2 / 0.015 | C-2 / 0.310 |
| TE-6 | P-6 | SM-2 / 30 | SM-3 / 57 | SM-7 / 13 | CA-1 / 0.400 | A-2 / 0.020 | C-1 / 0.025 |
| TE-7 | P-7 | SM-2 / 23 | SM-3 / 51 | SM-7 / 15 | CA-2 / 0.030 | CA-1 / 0.011 | C-1 / 0.025 |
| | | | SM-5 / 11 | | | | |
| CE-1 | R-1 | SM-1 / 27 | SM-3 / 55 | SM-6 / 18 | CA-3 / 0.020 | CA-1 / 0.094 | A-1 / 0.120 |

| | | | | | | | |
|---|---|---|---|---|---|---|---|
| In Table 1: TE: Test Example CE: Comparison Example Silicides are shown for "Type / Amount of use" Catalysts and surfactants are shown for "Type / Concentration" Amount of use: Molar % with respect to the total 100% of silanol group forming silicides used as material Concentration of catalyst for hydrolysis: Concentration (mass %) of catalyst with respect to silanol group forming silicide SiX₄ Concentration of catalyst for condensation reaction: Concentration (mass %) of catalyst with respect to the total of silanol group forming silicides used as material Concentration of surfactant: Concentration of surfactant (mass %) with respect to the total of silanol group forming silicides used as material SM-1: Tetraethoxy silane SM-2: Tetramethoxy silane SM-3: Methyltrimethoxy silane SM-4: Propyltributoxy silane SM-5: Phenyltrimethoxy silane SM-6: Dimethyldimethoxy silane SM-7: Methylphenylmethoxyethoxy silane CA-1: Sodium hydroxide CA-2: Ammonia CA-3: Acetic acid C-1: Lauryltrimethyl ammonium ethosulfate C-2: Tributylmethyl ammonium=diehtylphosphate A-1: Dodecylbenzene sodium sulfonate | | | | | | | |

**Table 2**

| | Type of particles | Siloxane units SiO₂ | | Siloxane units R¹SiO_{1.5} | | Siloxane units R²R³SiO | | Shape as a whole | Concave parts on surface | Hollow part |
|---|---|---|---|---|---|---|---|---|---|---|
| | | Type | Ratio | Type | Ratio | Type | Ratio | | | |
| TE-1 | P-1 | S-1 | 26 | S-2 | 53 | S-5 | 21 | 1* | 3* | 5* |
| TE-2 | P-2 | S-1 | 32 | S-2 | 54 | S-5 | 14 | 1* | 3* | 5* |
| TE-3 | P-3 | S-1 | 22 | S-2 | 62 | S-5 | 6 | 1* | 3* | 5* |
| | | | | S-3 | 10 | | | | | |
| TE-4 | P-4 | S-1 | 40 | S-2 | 44 | S-5 | 8 | 1* | 3* | 5* |
| | | | | S-4 | 8 | | | | | |
| TE-5 | P-5 | S-1 | 37 | S-2 | 51 | S-6 | 12 | 1* | 3* | 5* |
| TE-6 | P-6 | S-1 | 30 | S-2 | 57 | S-6 | 13 | 1* | 3* | 5* |
| TE-7 | P-7 | S-1 | 23 | S-2 | 51 | S-6 | 15 | 1* | 3* | 5* |
| | | | | S-4 | 11 | | | | | |
| CE-1 | R-1 | S-1 | 27 | S-2 | 55 | S-5 | 18 | 2* | 4* | 6* |

| | | | | | | | | | | |
|---|---|---|---|---|---|---|---|---|---|---|
| In Table 2: S-1: Anhydrous silicic acid unit S-2: Methyl siloxane unit S-3: Propyl siloxane unit S-4: Phenyl siloxane unit S-5: Dimethyl siloxane unit S-6: Methylphenyl siloxane unit Ratio: Molar % 1*: Spindle shape as a whole with a plurality of concave parts on the surface and a crack in the direction of the major axis 2* Shape of a rugby ball as a whole with a crack on peripheral surface in the longitudinal direction 3* Existence of a plurality of concave parts on the surface of fine particles 4* Fine particles have smooth surfaces 5* Existence of hollow part inside fine particles 6* No hollow part inside fine particles | | | | | | | | | | |

**Table 3**

| | Type of particles | Shape | | | Concave parts on surface | | Oil absorption (ml/100g) |
|---|---|---|---|---|---|---|---|
| | | (1) Average of major axes (L₁) in µm | (2) Average of minor axes (L₂) in µm | Ratio (2)/(1) | (3) Average of maximum diameters (m₁) of concave parts on surface in µm | Ratio (3)/(1) | |
| TE-1 | P-1 | 7.6 | 4.2 | 0.55 | 1.06 | 0.14 | 145 |
| TE-2 | P-2 | 10.3 | 5.4 | 0.52 | 2.16 | 0.21 | 125 |
| TE-3 | P-3 | 18.4 | 12.7 | 0.69 | 4.78 | 0.26 | 96 |
| TE-4 | P-4 | 0.8 | 0.3 | 0.39 | 0.06 | 0.08 | 67 |
| TE-5 | P-5 | 15.1 | 6.6 | 0.44 | 1.66 | 0.11 | 88 |
| TE-6 | P-6 | 13.6 | 5.6 | 0.41 | 3.67 | 0.27 | 92 |
| TE-7 | P-7 | 0.4 | 0.3 | 0.76 | 0.01 | 0.03 | 59 |
| CE-1 | R-1 | 2.5 | 1.2 | 0.48 | - | - | 39 |

### Part 2

In order to examine the utility of non-spherical hollow fine particles of this invention as cosmetic products, the following cosmetic products were prepared and evaluated.

### Preparation of cosmetic products

Compositions as shown in Table 4 were prepared. The preparation was made by using a mixer to mix pigments numbered 1-7 at the mass ratios shown in Table 4, and a mixture which had been separately prepared by taking the components preliminarily numbered 8-12 at the mass ratios shown in Table 4 and had been heated to 40°C was added thereinto and mixed again. After this mixture was left to be cooled, it was crushed and molded to be made the sample.

**Table 4**

| Number | Composition | Mass ratio |
|---|---|---|
| 1 | Fine particles which are objects of evaluation | 7 |
| 2 | Titanium oxide | 10 |
| 3 | Talc | 20 |
| 4 | Sericite | 35 |
| 5 | Red iron oxide | 0.45 |
| 6 | Yellow iron oxide | 1 |
| 7 | Black iron oxide | 0.05 |
| 8 | Fluidic paraffin | 10 |
| 9 | Octylmethyl cyclotetra siloxane | 5 |
| 10 | Polyoxyalkylene modified silicone | 1.5 |
| 11 | Sorbitan aliphatic ester | 5 |
| 12 | Myristyl alcohol | 5 |

### Evaluation

The sample described above was evaluated individually by twenty female panelists regarding its usability (extensions and expansions at the time of use) and feeling (stickiness, roughness and durability) according to the evaluation standards shown in Table 5. The results which have been rounded off are shown in Table 6.

**Table 5**

| Point | Evaluation standard |
|---|---|
| 4 | Very good |
| 3 | Good |
| 2 | Somewhat poor |
| 1 | Poor |

**Table 6**

| | Type of particles | Evaluation | | | |
|---|---|---|---|---|---|
| | | Extensions and expansions | Stickiness | Roughness | Durability |
| TE-8 | P-1 | 4 | 4 | 4 | 4 |
| TE-9 | P-2 | 4 | 4 | 4 | 4 |
| TE-10 | P-3 | 3 | 4 | 4 | 3 |
| TE-11 | P-4 | 4 | 3 | 4 | 3 |
| TE-12 | P-5 | 4 | 3 | 3 | 3 |
| TE-13 | P-6 | 3 | 4 | 3 | 3 |
| TE-14 | P-7 | 3 | 3 | 3 | 3 |
| CE-2 | R-1 | 3 | 2 | 3 | 2 |
| CE-3 | R-2 | 2 | 1 | 1 | 3 |
| CE-4 | R-3 | 1 | 1 | 1 | 2 |
| CE-5 | R-4 | 1 | 1 | 1 | 1 |

| | | | | | |
|---|---|---|---|---|---|
| In Table 6: R-2: Spherical silicone fine particles (TAK-100 (tradename) produced by Takemoto Yushi) R-3: Spherical vinyl fine particles (Ganz Pearl GSM (tradename) produced by Ganz Chemical Co., Ltd.) R-4: Talc | | | | | |

### Part 3

In order to examine the utility of non-spherical hollow fine particles of this invention as resin composition, polycarbonate resins with stronger requirements regarding optical and heat resistance characteristics were used for preparation and evaluation of the following resin compositions.

### Preparation of resin compositions and production of molded products

Non-spherical hollow fine particles of this invention (0.7 parts) were added to polycarbonate resin (Panlite K1285 (tradename) produced by Teijin Chemicals, Ltd.) (100 parts) and after they were mixed together, they were melted and kneaded together at resin temperature of 280°C by using a biaxial extruder (40mmΦ) equipped with vent to obtain pellets of resin composition by extrusion. Next, these pellets of resin composition were molded by using an injection molding machine at cylinder temperature of 230°C and mold temperature of 60°C to produce test pieces of 200 x 500mm with thickness 3mm.

### Evaluation

Optical characteristics (total light transmittance and haze) and heat-resistant colorability were measured as follows by using the test pieces described above.

Total light transmittance and haze were measured according to JIS-K7105 (1981) by using NDH-2000 (tradename) produced by Nippon Denshoku Industries Co., Ltd.

For the measurement of heat-resistant colorability, the aforementioned test piece was cut to produce 200 x 200mm sample films. The sample films thus cut out were placed inside a heated air circulating oven at temperature of 80°C and maintained there for 180 minutes. Thereafter, the degree of coloration by heating was measured in terms of the b-value by using a color meter (CR-300 (tradename) produced by Minolta Co., Ltd.). The value of Δb was calculated according to JIS-Z8729 (2004) for the formula Δb = b₂ - b₁ where b₁ is the b-value of the sample film before the heat treatment and b₂ is the b-value of the sample film after the heat treatment, and the calculated result is shown in Table 7.

As can be clearly understood from the results shown in Tables 6 and 7, non-spherical hollow fine particles of this invention are fully capable of responding to high levels of requirements as cosmetic products and resin compositions.

**Table 7**

| | Fine particles | Total light transmittance | Haze | Heat-resistant colorability |
|---|---|---|---|---|
| TE-15 | P-1 | 91.6 | 90.6 | -0.1 |
| TE-16 | P-2 | 90.8 | 92.1 | 0.0 |
| TE-17 | P-3 | 87.2 | 86.7 | 0.2 |
| TE-18 | P-4 | 85.5 | 85.7 | 0.2 |
| TE-19 | P-5 | 85.9 | 85.5 | 0.3 |
| TE-20 | P-6 | 86.8 | 84.8 | 0.2 |
| TE-21 | P-7 | 86.1 | 86.5 | 0.2 |
| CE-6 | R-1 | 81.6 | 80.3 | 0.2 |
| CE-7 | R-2 | 82.1 | 79.1 | 0.2 |
| CE-8 | R-3 | 80.8 | 79.6 | 3.9 |
| CE-9 | R-5 | 78.4 | 76.9 | 2.3 |

| | | | | |
|---|---|---|---|---|
| In Table 7: R-5: Calcium carbonate Total light transmittance: % Heat-resistant colorability: Value of Δb | | | | |

## Claims

1. Non-spherical hollow fine particles having a spindle shape as a whole with a major axis and a minor axis, a plurality of concave parts on a surface, a hollow part inside connected to the surface through a crack extending along the major axis, the average length of the major axes being 0.1-30µm and the ratio of the average length of the minor axes to the average length of the major axes being 0.3-0.8.

2. The non-spherical hollow fine particles of claim 1 wherein the average length of the major axes is 0.5-20µm.

3. The non-spherical hollow fine particles of claims 1 or 2 wherein the ratio of maximum diameters of the concave parts to the average length of the major axes is 0.01 - 0.30.

4. The non-spherical hollow fine particles of any of claims 1 to 3 of which oil absorption is 50 - 150ml/100g.

5. The non-spherical hollow fine particles of any of claims 1 to 4 comprising siloxane units SiO₂ in an amount of 20 - 45 molar %, siloxane units R¹SiO_{1.5} in an amount of 50 - 75 molar % and siloxane units R²R³SiO in an amount of 5 - 27 molar % so as to be a total of 100 molar %, wherein R¹, R² and R³ are each an organic group having a carbon atom directly connected to a silicon atom.

6. The non-spherical hollow fine particles of claim 5 wherein R¹, R² and R³ are each alkyl group with 1 - 4 carbon atoms or phenyl group.

7. A method of producing the non-spherical hollow fine particles of claim 5, said method comprising the steps of:
using silanol group forming silicide SiX₄ in an amount of 20 - 45 molar %, silanol group forming silicide R⁴SiY₃ in an amount of 50 - 75% and silanol group forming silicide R⁵R⁶SiZ₂ in an amount of 5 - 27 molar % so as to be a total of 100 molar %;
generating a silanol compound by causing silanol group forming silicide SiX₄ to contact water in the presence of a basic catalyst so as to undergo hydrolysis; and
causing a condensation reaction of said silanol compound with silanol group forming silicide R⁴SiY₃ and silanol group forming silicide R⁵R⁶SiZ₂ in an aqueous condition in the presence of a basic catalyst and a cationic surfactant;
wherein R⁴, R⁵ and R⁶ are each an organic group having a carbon atom directly connected to a silicon atom, and X, Y and Z are each alkoxy group with 1-4 carbon atoms, alkoxyethoxy group having alkoxy group with 1-4 carbon atoms, acyloxy group with 2-4 carbon atoms, N,N-dialkylamino group having alkyl group with 1-4 carbon atoms, hydroxyl group, halogen atom or hydrogen atom.

8. The method of claim 7 wherein R⁴, R⁵ and R⁶ are each alkyl group with 1 - 4 carbon atoms or phenyl group.

9. A cosmetic material containing the non-spherical hollow fine particles of any of claims 1 to 6.

10. A resin composition containing the non-spherical hollow fine particles of any of claims 1 to 6.

## Patentansprüche

1. Nicht-sphärische hohle feine Partikel, aufweisend insgesamt eine Spindelform mit einer Hauptachse und einer Nebenachse, eine Mehrzahl von konkaven Teilen auf einer Oberfläche, einen hohlen Teil im Inneren, verbunden mit der Oberfläche durch einen Spalt, sich erstreckend entlang der Hauptachse, wobei die mittlere Länge der Hauptachsen 0,1-30 µm beträgt und das Verhältnis der mittleren Länge der Nebenachsen zu der mittleren Länge der Hauptachsen 0,3-0,8 beträgt.

2. Nicht-sphärische hohle feine Partikel nach Anspruch 1, wobei die mittlere Länge der Hauptachsen 0,5-20 µm beträgt.

3. Nicht-sphärische hohle feine Partikel nach den Ansprüchen 1 oder 2, wobei das Verhältnis der maximalen Durchmesser der konkaven Teile zu der mittleren Länge der Hauptachsen 0,01-0,30 beträgt.

4. Nicht-sphärische hohle feine Partikel nach einem der Ansprüche 1 bis 3, deren Ölabsorption 50-150 ml/100 g beträgt.

5. Nicht-sphärische hohle feine Partikel nach einem der Ansprüche 1 bis 4, umfassend Siloxaneinheiten SiO₂ in einem Anteil von 20-45 Mol-%, Siloxaneinheiten R¹SiO_{1.5} in einem Anteil von 50-75 Mol-% und Siloxaneinheiten R²R³SiO in einem Anteil von 5-27 Mol-%, um insgesamt 100 Mol-% zu betragen, wobei R¹, R² und R³ jeweils eine organische Gruppe, aufweisend ein Kohlenstoffatom, direkt verbunden mit einem Siliciumatom, sind.

6. Nicht-sphärische hohle feine Partikel nach Anspruch 5, wobei R¹, R² und R³ jeweils eine Alkylgruppe mit 1-4 Kohlenstoffatomen oder eine Phenylgruppe sind.

7. Verfahren zum Herstellen der nicht-sphärischen hohlen feinen Partikel nach Anspruch 5, wobei das Verfahren die Schritte umfasst:
Verwenden des eine Silanolgruppe erzeugenden Silicids SiX₄ in einem Anteil von 20-45 Mol-%, des eine Silanolgruppe erzeugenden Silicids R⁴SiY₃ in einem Anteil von 50-75 Mol-% und des eine Silanolgruppe erzeugenden Silicids R⁵R⁶SiZ₂ in einem Anteil von 5-27 Mol-%, um insgesamt 100 Mol-% zu betragen;
Generieren einer Silanolverbindung, indem bewirkt wird, dass das eine Silanolgruppe erzeugende Silicid SiX₄ in Gegenwart eines basischen Katalysators mit Wasser in Kontakt kommt, um Hydrolyse durchzumachen; und
Bewirken einer Kondensationsreaktion der Silanolverbindung mit dem eine Silanolgruppe erzeugenden Silicid R⁴SiY₃ und dem eine Silanolgruppe erzeugenden Silicid R⁵R⁶SiZ₂ in einem wässerigen Zustand in Gegenwart eines basischen Katalysators und eines kationischen Tensids;
wobei R⁴, R⁵ und R⁶ jeweils eine organische Gruppe, aufweisend ein Kohlenstoffatom, direkt verbunden mit einem Siliciumatom, sind und X, Y und Z jeweils eine Alkoxygruppe mit 1-4 Kohlenstoffatomen, eine Alkoxyethoxygruppe, aufweisend eine Alkoxygruppe mit 1-4 Kohlenstoffatomen, eine Acyloxygruppe mit 2-4 Kohlenstoffatomen, eine N,N-Dialkylaminogruppe, aufweisend eine Alkylgruppe mit 1-4 Kohlenstoffatomen, eine Hydroxylgruppe, ein Halogenatom oder ein Wasserstoffatom sind.

8. Verfahren nach Anspruch 7, wobei R⁴, R⁵ und R⁶ jeweils eine Alkylgruppe mit 1-4 Kohlenstoffatomen oder eine Phenylgruppe sind.

9. Kosmetisches Material, enthaltend die nicht-sphärischen hohlen feinen Partikel nach einem der Ansprüche 1 bis 6.

10. Harzzusammensetzung, enthaltend die nicht-sphärischen hohlen feinen Partikel nach einem der Ansprüche 1 bis 6.

## Revendications

1. Particules fines creuses non sphériques ayant une forme de fuseau dans sa totalité avec un axe principal et un axe secondaire, une pluralité de parties concaves sur une surface, une partie creuse à l'intérieur reliée à la surface par une fissure s'étendant le long de l'axe principal, la longueur moyenne des axes principaux étant de 0,1-30 µm et le rapport de la longueur moyenne des axes secondaires sur la longueur moyenne des axes principaux étant de 0,3-0,8.

2. Particules fines creuses non sphériques selon la revendication 1, dans lesquelles la longueur moyenne des axes principaux est de 0,5-20 µm.

3. Particules fines creuses non sphériques selon la revendication 1 ou 2, dans lesquelles le rapport des diamètres maximaux des parties concaves sur la longueur moyenne des axes principaux est de 0,01-0,30.

4. Particules fines creuses non sphériques selon l'une quelconque des revendications 1 à 3, dont l'absorption d'huile est de 50-150 ml/100 g.

5. Particules fines creuses non sphériques selon l'une quelconque des revendications 1 à 4, comprenant des unités de siloxane SiO₂ dans une quantité de 20-45% molaire, des unités de siloxane R¹SiO_{1.5} dans une quantité de 50-75% molaire et des unités de siloxane R²R³SiO dans une quantité de 5-27% molaire de manière à être un total de 100% molaire, dans lesquelles R¹, R² et R³ sont chacun un groupe organique possédant un atome de carbone directement relié à un atome de silicium.

6. Particules fines creuses non sphériques selon la revendication 5, dans lesquelles R¹, R² et R³ sont chacun un groupe alkyle avec 1-4 atomes de carbone ou un groupe phényle.

7. Procédé pour la production des particules fines creuses non sphériques selon la revendication 5, ledit procédé comprenant les étapes:
d'utilisation d'un groupe silanol formant un siliciure SiX₄ dans une quantité de 20-45% molaire, d'un groupe silanol formant un siliciure R⁴SiY₃ dans une quantité de 50-75% molaire et d'un groupe silanol formant un siliciure R⁵R⁶SiZ₂ dans une quantité de 5-27% molaire de manière à être un total de 100% molaire;
de génération d'un composé de silanol en faisant entrer en contact le groupe silanol formant un siliciure SiX₄ avec de l'eau en présence d'un catalyseur basique de manière à être soumis à une hydrolyse; et
d'entraînement d'une réaction de condensation dudit composé de silanol avec un groupe silanol formant un siliciure R⁴SiY₃ et un groupe silanol formant un siliciure R⁵R⁶SiZ₂ dans une condition aqueuse en présence d'un catalyseur basique et d'un tensioactif cationique;
dans lequel R⁴, R⁵ et R⁶ sont chacun un groupe organique possédant un atome de carbone directement relié à un atome de silicium et X, Y et Z sont chacun un groupe alcoxy avec 1-4 atomes de carbone, un groupe alcoxyéthoxy possédant un groupe alcoxy avec 1-4 atomes de carbone, un groupe acyloxy avec 2-4 atomes de carbone, un groupe N,N-dialkylamino possédant un groupe alkyle avec 1-4 atomes de carbone, un groupe hydroxyle, un atome d'halogène ou un atome d'hydrogène.

8. Procédé selon la revendication 7, dans lequel R⁴, R⁵ et R⁶ sont chacun un groupe alkyle avec 1-4 atomes de carbone ou un groupe phényle.

9. Matériau cosmétique contenant les particules fines creuses non sphériques selon l'une quelconque des revendications 1 à 6.

10. Composition de résine contenant les particules fines creuses non sphériques selon l'une quelconque des revendications 1 à 6.
